# FASCICULE DE BREVET EUROPEEN

(11) **EP 0 676 187 B1**
(45) Date de publication et mention de la délivrance du brevet: **27.04.2005**
(21) Numéro de dépôt: 95400363.8
(22) Date de dépôt: 21.02.1995
(51) Int. Cl.: A61K 7/00, A61K 7/48, A61K 33/30

(54) **Composition contenant un oxyde de métal non photocatalytique et du tocophérol et son utilisation dans le domaine dermatologique**
Zusammensetzung enthaltend ein nicht photokatalytisches Metalloxid und Tocopherol und ihre dermatologische Verwendung
Composition containing a non photocatalytic metal oxide and tocopherol and its dermatological use

(30) Priorité: 11.03.1994 FR 9402882
(43) Date de publication de la demande: 11.10.1995
(73) Titulaire: L'OREAL, 75008 Paris (FR)
(72) Inventeur: Boussouira, Boudiaf, F-75013 Paris (FR); Nguyen, Quang Lan, F-92160 Antony (FR)
(74) Mandataire: Lhoste, Catherine

(56) Documents cités:
- EP-A- 0 280 606
- EP-A- 0 353 161
- EP-A- 0 535 446
- EP-A- 0 579 078
- WO-A-84/02845
- WO-A-92/01439
- WO-A-92/17160
- WO-A-92/19224
- WO-A-93/14773
- CD-ROM VIDAL 1992, O.V.P. - EDITIONS DU VIDAL, PARIS., XP002033476
- CD-ROM VIDAL 1992, O.V.P. - EDITIONS DU VIDAL, PARIS., XP002033477
- DATABASE WPI Section Ch, Week 9206 Derwent Publications Ltd., London, GB; Class A17, AN 92-042074 XP002033478 & DD 293 477 A (BUNA AG) , 5 septembre 1991

## Description

La présente invention se rapporte à une composition contenant au moins un tocophérol choisi parmi l'α-tocophérol, le β-tocophérol, le γ-tocophérol, et/ou le δ-tocophérol, au moins un oxyde de métal non photocatalytique et au moins un agent complexant inactivateur de métaux choisi parmi les dérivés d'acide phosphonique et l'utilisation de cette composition pour la fabrication d'une composition destinée au traitement dermatologique de lutte contre et/ou de prévention de l'irritation, de l'inflammation, de l'immunosuppression et/ou l'acné photoinduits. Cette composition permet en particulier l'inhibition de la peroxydation photoinduite de lipides insaturés, et plus spécialement de lipides d'origine sébacée (sébum).

La composition de l'invention se présente, en particulier, sous forme d'une crème pouvant être appliquée aussi bien sur le visage que sur le cuir chevelu et les cheveux, ainsi que sur le corps humain. Elle peut aussi servir de base à un rouge-à-lèvres.

De façon plus précise, l'invention a pour objet une composition contenant un oxyde de zinc et du tocophérol agissant de manière synergique.

On sait que les lipides se trouvant à la surface de la peau, du cuir chevelu et des cheveux sont soumis en permanence à des agressions extérieures et notamment l'air, les polluants atmosphériques et les rayonnements visibles et surtout ultraviolets (UV).

Ces lipides sont aussi bien ceux qui font partie des constituants de la peau ou des cheveux, que ceux qui sont sécrétés par la peau y compris le cuir chevelu et/ou ceux qui sont déposés sur la peau ou les cheveux lors de l'application sur ces dernière de produits contenant des lipides.

Les lipides les plus exposés aux agressions extérieures sont ceux contenus dans les sécrétions grasses de la peau comme le sébum, riche en squalène. La présence dans les molécules de squalène de six doubles liaisons rend ces molécules sensibles aux phénomènes d'oxydation. Ainsi, lors d'une exposition prolongée aux UV, le squalène se photoperoxyde pour donner des peroxydes de squalène.

Cette production élevée de peroxydes de squalène, provoque notamment une série de dégradations en chaîne en particulier dans et sur la peau, donnant naissance à de nombreux désordres cutanés. Ainsi ces peroxydes de squalène interviennent notamment dans :
- la pathogénèse de l'acné comme décrit par Saint Léger et al. (voir British Journal of Dermatology, 1986 : 114, pp 535-542) qui précisent que les peroxydes de squalène sont comédogènes ;
- le vieillissement prématuré de la peau comme décrit par Keiko OH Sawa et al. (voir The Journal of Toxicology Sciences, 1984 : 19, pp 151-159) qui traitent des conséquences des brûlures cutanées dues au soleil ;
- les phénomènes d'irritation comme rapportés par Takayoshi Tanaka et al. (voir J.Clin.Biochem.Nutr., 1986 : 1, pp 201-207) qui signalent les dommages provoqués notamment par l'utilisation répétée de certains shampooings ;
- la production de produits volatils malodorants (aldéhydes, cétones, acides, etc) et enfin dans
- l'immunosuppression, comme messagers biochimiques des effets biologiques de l'irridiation UV de la peau, comme décrit par M. PICARDO et al. (voir Photodermatol. Photoimmunol. Photomed., 1991:8, pp 105-110).

Afin de limiter la peroxydation des lipides insaturés, il est connu d'appliquer sur la peau des compositions photoprotectrices renfermant au moins un agent anti-radicaux libres et au moins un agent filtrant.

C'est le cas, par exemple, de la composition complexe décrite dans le document FR-A-2666226, relative à une crème contre les intolérances aux photons, et qui renferme parmi d'autres constituants anti-radicaux libres, l'alpha-tocophérol et parmi plusieurs filtres physiques et chimiques présents dans ladite composition, l'oxyde de titane.

Cette composition présente l'inconvénient d'être peu protectrice contre les rayonnements ultraviolets et d'être inappropriée aux domaines cosmétique et dermatologique en raison des problèmes liés à l'activité "photocatalytique" de l'oxyde de titane comme expliqué ci-après.

En effet, la demanderesse a mis en évidence, selon la méthode "Head space", que l'oxyde de titane, sous exposition ultraviolette ou après quelques heures à 37° C, catalyse la production de radicaux peroxydes à partir des constituants lipidiques contenus dans les compositions cosmétiques. Ceci entraîne, lors de l'application sur la peau de ces compositions, des effets nocifs tels que des inflammations. Pour une description de cette méhode, on peut se référer en particulier aux articles de N'GUYEN QL et al. Symposium of AFECG-SFC, Bordeaux mai 1984, pp 358-359, Evaluation de l'oxydation aldéhydique dans les produits cosmétiques ; et de WARNER K et Coll. Journal of Food Science- 1974 V39- pp 761-765, "Pentane formation and rancidity in vegetable oils".

C'est pourquoi, pour surmonter les inconvénients liés à l'utilisation d'oxyde de titane, il a été suggéré par exemple dans le document WO-A-9009777 des traitements de surface de l'oxyde de titane. Toutefois de tels traitements ne permettent pas de diminuer de manière suffisante l'activité photocatalytique de cet oxyde et l'utilisation de ce dernier, traité, peut encore provoquer des dommages de la peau lors d'une exposition aux UV.

On connaît, par ailleurs, les propriétés bénéfiques de l'oxyde de zinc pour la peau. En particulier, il retient les graisses et l'humidité, absorbe les UV, possède un bon pouvoir couvrant et est, en outre, apaisant (voir par exemple l'article de L.D GRADY paru dans The Journal of the Society of Cosmetic Chemistry : 1947, Juillet, volume 1). Ainsi l'oxyde de zinc a pu être incorporé dans des compositions cosmétiques comme la poudre pour le visage (voir l'article ci-dessus de L.D GRADY).

Plus récemment, la Société NIVEA a commercialisée une composition, sous forme de masque, dénommée "Masque 3 minutes Hydro-purifiant" qui renferme, entre autres ingrédients, de l'oxyde de zinc, pour ses propriétés astringentes, de l'acétate de tocophérol, pour son activité dans la régénération cellulaire et du kaolin pour absorber l'excès de sébum.

Cette composition n'est pas prévue pour inhiber la photoperoxydation des lipides et notamment du squalène et n'est pas susceptible d'inhiber cette photoperoxydation.

Ainsi, les compositions connues à ce jour ne confèrent qu'une protection insuffisante, voire nulle, de la peau vis-à-vis de la peroxydation des lipides de, et sur, la peau.

On constate donc qu'il subsiste le besoin d'une composition capable d'inhiber les formes actives de l'oxygène, et notamment présentant une activité antilipoperoxydante efficace, notamment antiperoxydation du squalène, lors d'expositions ultraviolettes et/ou aux autres facteurs d'oxydation, qui soit bien tolérée par la peau et/ou les cheveux.

On entend par "formes actives de l'oxygène" les formes de l'oxygène, radicalaires ou non, comme notamment ROOH (R, représentant, en particulier, la chaîne hydrocarbonée d'un lipide insaturé), OH°, ROO°, O₂H°, ¹O₂, et plus particulièrement, ROOH, où R est la chaîne hydrocarbonée du squalène.

La demanderesse a maintenant découvert une composition permettant d'atteindre ces objectifs, contenant au moins un oxyde de métal non photocatalytique, au moins un tocophérol choisi parmi l'α-tocophérol, le β-tocophérol, le γ-tocophérol, et/ou le δ-tocophérol, pour inhiber la photoperoxydation des lipides insaturés et au moins un agent complexant inactivateur de métaux choisi parmi les dérivés d'acide phosphonique.

On connaît, du document GB-A-2184356, une crème antisolaire à base d'oxyde de zinc et d'une dizaine d'autres constituants dont un antioxydant. Mais ce document n'enseigne pas d'utiliser, comme antioxydant, un tocophérol en vue de lutter contre la photoperoxydation de lipides insaturés notamment d'origine sébacée, comme le squalène.

Par ailleurs, il est connu du document EP-A-579078 d'associer de l'oxyde de zinc et du tocophérol, mais ce document n'enseigne ni ne suggère l'inhibition de la photoperoxydation des lipides insaturés par cette association, d'autant que, dans les exemples, le tocophérol est utilisé sous forme d'acétate, forme qui n'est pas susceptible d'inhiber cette photoperoxydation.

Or, la demanderesse a pu démontrer, grâce à ses recherches intensives dans le domaine des compositions inhibitrices de la peroxydation du sébum, que l'efficacité de telles compositions dépend du choix de l'antioxydant, à associer à l'oxyde de métal non photocatalytique.

Ainsi l'invention a encore pour objet l'utilisation du système de l'invention pour la fabrication d'un produit destiné au traitement dermatologique de lutte contre, et/ou de prévention de, l'irritation, l'inflammation, l'immunosuppression et/ou l'acné, qui sont induits par la photoperoxydation, en particulier, du squalène.

On entend par agent complexant inactivateur de métaux un agent qui évite la formation des radicaux hydroxyle OH° par suite d'une forte capture des métaux présents dans la composition, lorsque ces agents sont soumis à un rayonnement, notamment UV. En particulier, ces agents assurent la complexation sous UV, des métaux contenus dans l'eau, et notamment du fer.

En particulier, la demanderesse a observé que le butylhydroxytoluène (BHT), la superoxydedismutase (SOD) qui sont des antioxydants notoires, n'ont qu'un faible effet antilipoperoxydant et ne peuvent donc pas être utilement associés à un oxyde de métal non photocatalytique pour inhiber la peroxydation des lipides insaturés.

Selon l'invention, la composition ne présente aucun risque d'intolérance et est utilisable en toute sécurité, notamment dans les domaines, dermatologique, voire encore vétérinaire.

En outre, elle procure une protection efficace vis-à-vis de toute agression oxydative sans avoir recours à d'autres filtres, notamment chimiques. Ainsi, la composition de l'invention possède un côté plus naturel, recherché par le consommateur, que les compositions existantes dans le domaine cosmétique, et est beaucoup plus simple à réaliser que ces compositions, donc moins coûteuse.

La composition de l'invention peut se présenter sous diverses formes galéniques, et en particulier sous forme d'émulsion huile-dans-eau ou eau-dans-huile, de solutions, de gels ou de dispersions vésiculaires. Cette composition peut être une crème de soin, un shampooing, une lotion, ou un sérum.

Le rapport pondéral oxyde de métal non photocatalytique/tocophérol, dans la composition de l'invention, est choisi en particulier de 100/1 à 1/4, et de préférence de 10/1 à 2/1.

Selon l'invention, l'oxyde de métal non photocatalytique utilisable dans la composition est, de préférence, un oxyde de métal bivalent.

Cet oxyde de métal peut être enrobé ou non.

Comme oxyde de métal bivalent non photocatalytique, on peut utiliser les oxydes de zinc, les oxydes de titane traités, les oxydes de sélénium, les oxydes de cérium, les oxydes de magnésium, et les oxydes de zirconium. De préférence, on utilise des oxydes de zinc.

De plus, l'oxyde de zinc est présent dans la composition de l'invention, de préférence, sous forme de particules sphériques. Avantageusement, il ne doit pas contenir de traces de métaux lourds nocifs tels que le cadmium, le plomb, etc. Le diamètre moyen de ces nanoparticules est choisi, par exemple, de 1 nm à 500 nm, et de préférence de 100 nm.

Comme oxyde de zinc, on peut utiliser celui décrit dans le document WO-A-9213517 et commercialisé par la Société SUNSMART, sous la dénomination Z-cote. Ce type d'oxyde de zinc est transparent, ce qui est un avantage d'un point de vue esthétique, les filtres pulvérulents usuels étant généralement opaques.

L'oxyde de métal est généralement présent à raison de 0,1 % à 5 %, et de préférence de 0,5 % à 2 % en poids par rapport au poids total de la composition.

Selon l'invention, le tocophérol est présent dans la composition à " l'état libre ", c'est-à-dire, sans groupement additionnel et notamment sans groupement formant ester.

De préférence, le tocophérol utilisé est un mélange de tocophérols naturels, d'α-tocophérol, de β-tocophérol, de γ-tocophérol, et de δ-tocophérol ; ce mélange peut être utilisé notamment dans une huile choisie parmi les huiles végétales, minérales, siliconées, et de préférence végétales.

Comme mélange de tocophérols naturels utilisable selon l'invention, on peut citer celui dissous à 50 % dans l'huile de soja, vendu par la Société BIZEN sous la dénomination D mixed tocopherols. Le D α-tocophérol vendu par la Société HENKEL sous la dénomination Cophérol F1300 ou encore ceux décrits dans le document US-A-4144325 peuvent aussi être utilisés.

Le tocophérol (ou mélange) peut être présent (matière active) dans la composition de l'invention, en particulier à raison de 0,0005 % à 10 % et, de préférence à raison d'au moins 0,02 % et jusqu'à 6 % en poids par rapport au poids total de la composition.

L'agent complexant inactivateur de métaux utilisable dans la composition de l'invention est, un dérivé de l'acide phosphonique et est choisi en particulier parmi l'acide 3-éthylènediamine tétra(méthylène phosphonique), l'acide diéthylènetriamine penta(méthylène phosphonique) et leur sel de sodium. De préférence, l'agent complexant est le sel pentasodique de l'acide diéthylènetriamine penta(méthylènephosphonique).

Les agents complexants, comme l'éthylène diamine tétra-acétique (EDTA) qui est un chélateur du fer, possèdent un effet pro-oxydant sous rayonnement UV et ne peuvent donc pas être utilisés seuls dans la composition de invention à visée antioxydante. Toutefois, il est possible d'utiliser ce type d'agent en association avec un dérivé d'acide phosphonique.

Lorsque l'agent complexant inactivateur de métaux est présent, on l'utilise à une concentration qui est choisie de 0,005 % à 0,1 % en poids par rapport au poids total de la composition.

Aussi, une composition préférée selon l'invention pour inhiber les formes active de l'oxygène, comprend, en poids :
. de 0,05 % à 2 % d'un mélange de tocophérols naturels,
. de 0,5 % à 5 % d'oxyde de zinc et
. de 0,05 % à 0,1 % de sel de sodium de l'acide diéthylènetriamine penta (méthylène phosphonique).

La composition de l'invention peut par ailleurs contenir des adjuvants, utilisés seuls ou en mélange, et en particulier ceux choisis parmi les agents tensio-actifs (émulsionnant, ou coémulsionnant), de type non-ionique, anionique, cationique ou amphotère, les agents traitants, les actifs, les épaississants, les agents de mise en suspension, les colorants, les parfums, les charges, les agents neutralisants, les excipients (huiles/eau) et les conservateurs.

Les adjuvants cités peuvent être incorporés aux doses habituelles communément admises en évitant, dans la mesure du possible, ceux susceptibles de relarguer des métaux catalyseurs d'oxydation. lls peuvent être lipophiles ou hydrophiles.

Dans la composition selon l'invention, on peut utiliser avantageusement comme agent épaississant le polymère à base de polyacrylamide, d'isoparaffine en C₁₃-C₁₄ et de laureth-7 (selon la nomenclature CTFA).

La composition de l'invention est plus spécialement destinée à inhiber la peroxydation, induite par les rayonnements UV, des lipides insaturés du sébum, comme le squalène.

De manière surprenante, la demanderesse a découvert que la composition de l'invention inhibe très efficacement la formation de peroxydes de squalène induite par les UV en raison d'un effet surprenant de synergie entre ses deux constituants, l'oxyde de métal non photocatalytique et le tocophérol. Cet effet a été mis en évidence lors de tests "ex vivo" qui seront décrits en détails ultérieurement .

Dans le cas particulier du traitement de l'acné, on peut également incorporer dans la composition de l'invention, de façon avantageuse, un agent antiacnéïque spécifique, antiséborrhéique et/ou antibactérien, et en particulier, la piroctone olamine, vendue sous la dénomination Octopirox par la société HOECHST.

De par ses propriétés bénéfiques, la composition de l'invention convient à la protection de tout type de peaux et plus spécialement des peaux grasses et des peaux dites sensibles. Elle peut aussi servir à protéger les lèvres des gerçures.

D'autres caractéristiques et avantages de la composition de l'invention ressortiront mieux de la description, des exemples et contre-exemples. Dans ces exemples et contre-exemples, les pourcentages sont donnés en poids.

### Exemple 1 : Crème antiacnéïque pour peaux grasses

| | |
|---|---|
| Tocophérol et huile de soja (50/50) | 0,5 % |
| Stéarate de glycéryle | 0,3 % |
| FD et C Red No.4 (colorant) | 0,0001 % |
| Acid Yellow 3 (colorant) | 0,0005 % |
| Parahydroxybenzoate de méthyle (conservateur) | 2 % |
| Hexamidine diisethionate (conservateur) | 0,03 % |
| Piroctone olamine (antiacné) | 0,2 % |
| Parfum | 0,3 % |
| Triéthanolamine (neutralisant) | 0,002 % |
| Sel pentasodique de l'acide éthylènediamine tétra(méthylène phosphonique) | 0,01 % |
| Oxyde de zinc | 2 % |
| Gomme de xanthane (épaississant) | 0,3 % |
| Polyacrylamide/C13-C14 Isoparaffin/Laureth-7 (épaississant) | 2 % |
| Cyclométhicone (huile) | 6 % |
| Glycérol (actif) | 3 % |
| Propylèneglycol (actif) | 6 % |
| Alcool cétylique (coémulsionnant) | 1,0 % |
| Stéarate de PEG-20 (émulsionnant) | 1,7 % |
| D-Panthénol (actif) | 1 % |
| Eau quantité suffisante pour | 100 % |

La crème ainsi préparée a un pH de valeur 6 à 7 et protège la peau, et en particulier les constituants lipidiques à la surface de la peau, vis-à-vis des méfaits des UV. Elle peut être appliquée chaque matin.

### Contre-Exemple 1 :

On prépare une crème de même composition que celle de l'exemple 1 à l'exception de l'oxyde de zinc.

### Contre-Exemple 2 :

On prépare une crème de même composition que celle de l'exemple 1 à l'exception du tocophérol.

### Contre-Exemple 3 :

On prépare une crème de même composition que celle de l'exemple 1 à l'exception de l'oxyde de zinc et du tocophérol (placébo).

Pour mesurer l'efficacité respective de la composition de l'invention (Ex. 1), ainsi que celle des compositions des contre-exemples ci-dessus, on effectue un test Ex Vivo selon le protocole suivant :

On réalise un prélèvement de sébum sur le front de volontaires à l'aide de quatre papiers filtres hydrophiles de 17,4cm². On dépose ensuite sur un de ces quatre papiers filtres à raison de 4mg/cm² de la composition selon l'exemple 1. On applique ensuite respectivement sur le second, troisième et quatrième filtres environ 4mg/cm² des compositions du contre-exemple 1, du contre-exemple 2 et du placébo (sans oxyde de zinc ni tocophérol). On soumet le tout à une dose d'UVA de 5 joules par cm² pendant 50 min. La source UV utilisée a une puissance 2mW/cm².

Les résultats obtenus sont portés dans le tableau ci-après. Il indique les pourcentages d'inhibition de la photoperoxydation obtenus respectivement pour l'exemple 1 et les contre-exemples.

| **Ex.1(Invention)** | **Contre-Ex.1** | **Contre-Ex.2** | **Contre-Ex.3** |
|---|---|---|---|
| 65 % | 9 % | 18 % | 0 % |

On constate donc d'après ce tableau, que le niveau d'inhibition atteint par la composition de l'invention (65 %) est nettement supérieur à la somme des niveaux d'inhibition (9 % + 18 %) obtenus à l'aide de tocophérol et d'oxyde de zinc pris séparément. Il s'ensuit que le tocophérol augmente de façon inattendue l'efficacité de l'oxyde de zinc et réciproquement.

### Contre - Exemple 2 : Shampooing non-irritant

| | |
|---|---|
| Oxyde de zinc | 0,5 % |
| Carbomer(*) | 0,2 % |
| triéthanolamine (neutralisant) | 0,2 % |
| MEA-Laureth sulfate (**) (matière active) | 10 % |
| Coco bétaine (**) | 2 % |
| Cocamide DEA (**) | 3 % |
| Tocophérol et huile de soja (50/50) | 0,2 % |
| Parfum | qsp |
| Eau quantité suffisante pour | 100 % |

| | |
|---|---|
| (*) Carbopol 980, vendu par la société Goodrich. | |
| (**) Selon la nomenclature CTFA. | |

### Exemple 3 : Gel protecteur pour peaux sensibles

| | |
|---|---|
| Tocophérol et huile de soja | 0,5 % |
| FD et C Red No.4 | 0,0001 % |
| Acid Yellow 3 | 0,0005 % |
| Parahydroxybenzoate de méthyle | 0,18 % |
| Hexamidine diisethionate | 0,03 % |
| Parfum | 0,3 % |
| Triéthanolamine | 0,002 % |
| Sel pentasodique de l'acide éthylènediamine tétra(méthylène phosphonique) | 0,01 % |
| Oxyde de zinc | 2 % |
| Gomme de xanthane | 0,3 % |
| Polyacrylamide/C13-C14 Isoparaffin/Laureth-7 | 2 % |
| Cyclométhicone | 6 % |
| Glycérol | 3 % |
| Propylèneglycol | 6 % |
| D-Panthénol | 1 % |
| Eau quantité suffisante pour | 100 % |

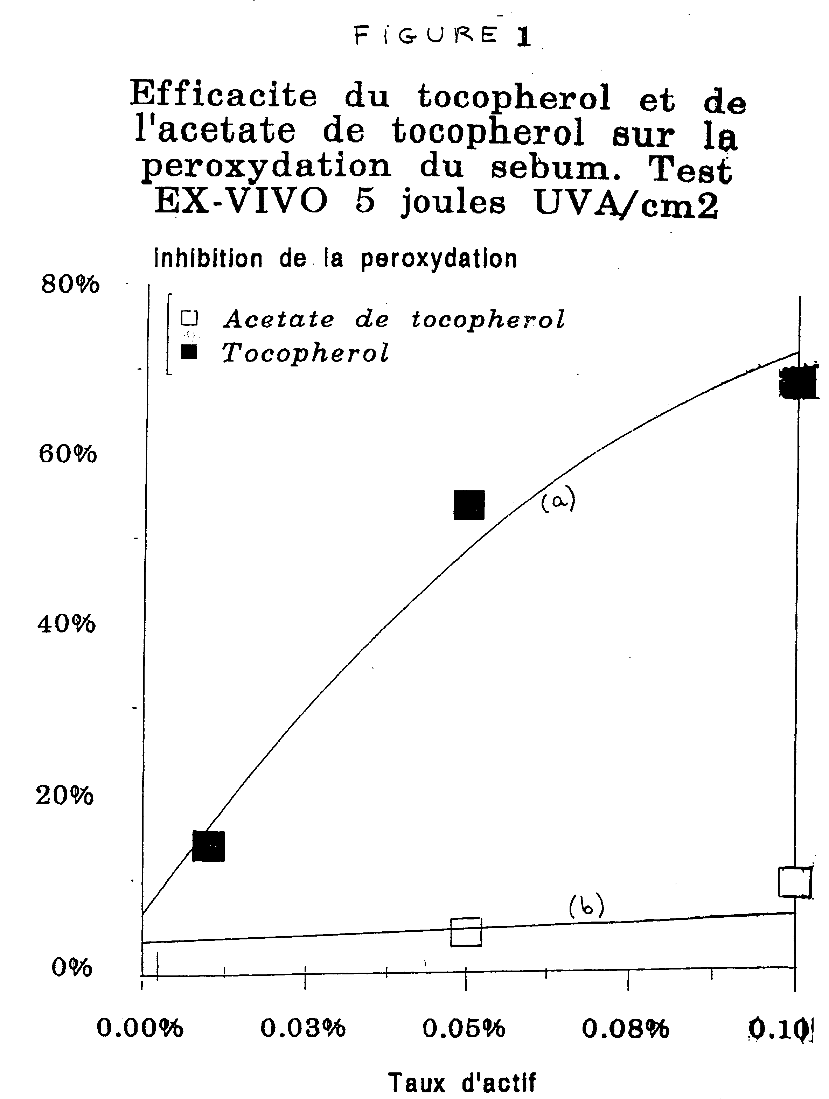

On présente ci-après un test *ex vivo* montrant la nécessité d'utiliser un tocophérol libre et non un dérivé de tocophérol tel que l'acétate de tocophérol. La figure 1 donne les courbes d'inhibition de la peroxydation du sébum. L'axe des ordonnées correspond au pourcentage d'inhibition de la peroxydation et l'axe des abscisses correspond à la concentration en tocophérol ou acétate de tocophérol. La courbe (a) correspond au tocophérol et la courbe (b) à l'acétate de tocophérol. En comparant ces deux courbes, il apparaît clairement que seul le tocophérol permet d'obtenir une bonne inhibition de la peroxydation du sébum.

Le protocole de test a été le suivant :
- Pour chaque courbe (a) et (b), on a prélevé du sébum sur le front d'un individu à l'aide de deux filtres hydrophiles ;
- dans chaque cas, on a appliqué sur un filtre un placebo et sur l'autre la crème contenant l'actif, c'est-à-dire le tocophérol dans un cas, l'acétate de tocophérol dans l'autre cas, ledit actif étant présent dans la crème en une concentration déterminée, le placebo et la crème étant appliqués en une concentration de 3 mg/cm² ;
- puis on a soumis les filtres à une irradiation de 5 joules UVA par cm² ;
- on a ensuite déterminé le taux de peroxydes de squalène sur les filtres par chromatographie en phase inverse et chimiluminescence.
- les résultats obtenus ont permis de tracer les courbes représentées sur la figure 1.

Les crèmes et le placebo ont les compositions indiquées ci-dessous :

| Composition | Placebo | Crème à 0,05 % | Crème à 0,1 % |
|---|---|---|---|
| Phase huileuse | 35 % | 34,95 % | 34,9 % |
| Actif | 0 % | 0,05 % | 0,1 % |
| Eau | 65 % | 65 % | 65 % |

La phase huileuse a la composition centésimale suivante :
- Alcool cétylique 14 %
- Stéarate de glycéryle 9 %
- Stéarate de PEG-50 9 %
- Triglycérides caprilique/caprique 9 %
- Huile minérale qsp 100 %

## Revendications

1. Utilisation d'un système formé d'au moins un oxyde de métal non photocatalytique, d'au moins un tocophérol choisi parmi l'α-tocophérol, le β-tocophérol, le γ-tocophérol, et/ou le δ-tocophérol, et d'un agent complexant inactivateur de métaux choisi parmi les dérivés d'acide phosphonique, pour la fabrication d'une composition destinée au traitement dermatologique de lutte contre et/ou de prévention de l'irritation, de l'inflammation, de l'immunosuppression et/ou l'acné photoinduits.

2. Utilisation selon la revendication 1, dans laquelle l'agent complexant est présent à raison de 0,005% à 0,1% en poids par rapport au poids total de la composition.

3. Utilisation selon l'une des revendications précédentes, **caractérisée en ce que** le dérivé d'acide phosphonique est choisi parmi l'acide éthylènediamine tétra(méthylène phosphonique), l'acide diéthylènetriamine penta(méthylène phosphonique) et leur sel de sodium.

4. Utilisation selon l'une quelconque des revendications précédentes, **caractérisée en ce que** le rapport pondéral oxyde de métal non photocatalytique/tocophérol est choisi de 100/1 à 1/4, notamment de 10/1 à 2/1.

5. Utilisation selon l'une quelconque des revendications précédentes, **caractérisée en ce que** l'oxyde de métal non photocatalytique est un oxyde de métal bivalent.

6. Utilisation selon la revendication 5 **caractérisée en ce que** l'oxyde de métal bivalent est l'oxyde de zinc.

7. Utilisation selon la revendication 6, **caractérisée en ce que** l'oxyde de zinc est sous forme de particules sphériques dont le diamètre moyen est de 1 nm à 500 nm.

8. Utilisation selon l'une quelconque des revendications précédentes, **caractérisée en ce que** l'oxyde de métal non photocatalytique est présent à raison de 0,5% à 2% en poids par rapport au poids total de la composition.

9. Utilisation selon l'une quelconque des revendications précédentes, **caractérisée en ce que** le tocophérol est présent à raison de 0,0005% à 10% en poids, notamment 0,02% à 6% en poids, par rapport au poids total de la composition.

10. Utilisation selon l'une quelconque des revendications précédentes, **caractérisée en ce que** le tocophérol est un mélange de tocophérols naturels.

11. Utilisation selon l'une quelconque des revendications précédentes, **caractérisée en ce que** le système contient en outre des adjuvants, utilisés seuls ou en mélange, choisis parmi les agents tensio-actifs de type nonionique, anionique, cationique ou amphotère, les agents traitants, les actifs, les épaississants, les agents de mise en suspension, les colorants, les parfums, les charges, les agents neutralisants, les excipients et les conservateurs.

12. Utilisation selon la revendication 11, **caractérisée en ce que** l'adjuvant est un agent antiacnéïque.

13. Utilisation selon l'une quelconque des revendications précédentes, **caractérisée en ce que** le système est incorporé dans une composition se présentant sous forme d'une crème ayant un pH de valeur 6-7 comprenant un polymère à base de polyacrylamide, d'isoparaffine en C₁₃-C₁₄ et de laureth-7.

14. Composition **caractérisée en ce qu'**elle comprend, dans un milieu cosmétiquement et/ou dermatologiquement acceptable, au moins un tocophérol choisi parmi l'α-tocophérol, le β-tocophérol, le γ-tocophérol, et/ou le δ-tocophérol, au moins un oxyde de métal non photocatalytique et au moins un agent complexant inactivateur de métaux choisi parmi les dérivés d'acide phosphonique.

15. Composition selon la revendication 14, **caractérisée en ce que** ledit agent complexant inactivateur de métaux est présent à raison de 0,005 % à 0,1 % en poids par rapport au poids total de la composition.

16. Composition selon l'une des revendications 14 à 15, **caractérisée en ce que** le dérivé d'acide phosphonique est choisi parmi l'acide éthylènediamine tétra(méthylène phosphonique), l'acide diéthylènetriamine penta(méthylène phosphonique) et leur sel de sodium.

17. Composition selon l'une des revendications 14 à 16, **caractérisée en ce que** le rapport pondéral oxyde de métal non photocatalytique/tocophérol est choisi de 100/1 à 1/4, notamment de 10/1 à 2/1.

18. Composition selon l'une des revendications 14 à 17, **caractérisée en ce que** l'oxyde de métal non photocatalytique est un oxyde de métal bivalent.

19. Composition selon la revendication 18, **caractérisée en ce que** l'oxyde de métal bivalent est l'oxyde de zinc.

20. Composition selon la revendication 19, **caractérisée en ce que** l'oxyde de zinc est sous forme de particules sphériques dont le diamètre moyen est de 1 nm à 500 nm.

21. Composition selon l'une des revendications 14 à 20, **caractérisée en ce que** l'oxyde de métal non photocatalytique est présent à raison de 0,1 à 5% en poids, notamment 0,5% à 2% en poids par rapport au poids total de la composition.

22. Composition selon l'une des revendications 14 à 21, **caractérisée en ce que** te tocophérol est présent à raison de 0,0005% à 10% en poids, notamment 0,02% à 6% en poids, par rapport au poids total de la composition.

23. Composition selon l'une des revendications 14 à 22, **caractérisée en ce que** le tocophérol est un mélange de tocophérols naturels.

24. Composition selon l'une des revendications 14 à 23, **caractérisée en ce qu'**elle contient en outre des adjuvants, utilisés seuls ou en mélange, choisis parmi les agents tensio-actifs de type non-ionique, anionique, cationique ou amphotère, les agents traitants, les actifs, les épaississants, les agents de mise en suspension, les colorants, les parfums, les charges, les agents neutralisants, les excipients et les conservateurs.

25. Composition selon la revendication 24, **caractérisée en ce que** l'adjuvant est un agent antiacnéïque.

26. Composition selon la revendication 14, **caractérisée en ce qu'**elle comprend, de 0,05% à 2% d'un mélange de tocophérols naturels, de 0,5% à 5% d'oxyde de zinc et de 0,05% à 0,1% de sel de sodium de l'acide diéthylènetriamine penta-(méthylène phosphonique).

## Patentansprüche

1. Verwendung eines Systems, das aus mindestens einem nicht photokatalytischen Metalloxid, mindestens einem Tocopherol, das unter α-Tocopherol, β-Tocopherol, γ-Tocopherol und/oder δ-Tocopherol ausgewählt ist, und einem komplexierenden, Metalle inaktivierenden Stoff, der unter den Phosphonsäurederivaten ausgewählt ist, gebildet wird, für die Herstellung einer Zusammensetzung, die für die dermatologische Behandlung zur Bekämpfung und/oder Vorbeugung von Irritationen, Entzündungen, Immunsupression und/oder lichtinduzierter Akne vorgesehen ist.

2. Verwendung nach Anspruch 1, wobei der Komplexbildner in einer Menge von 0,005 bis 0,1 Gew.-%, bezogen auf das Gesamtgewicht der Zusammensetzung, enthalten ist.

3. Verwendung nach einem der vorhergehenden Ansprüche, **dadurch gekennzeichnet, dass** das Phosphonsäurederivat unter Ethylendiamintetra(methylenphosphonsäure), Diethylentriaminpenta(methylenphosphonsäure) und deren Natriumsalzen ausgewählt ist.

4. Verwendung nach einem der vorhergehenden Ansprüche, **dadurch gekennzeichnet, dass** das Gewichtsverhältnis nicht photokatalytisches Metalloxid/Tocopherol im Bereich von 100:1 bis 1:4 und insbesondere 10:1 bis 2:1 ausgewählt ist.

5. Verwendung nach einem der vorhergehenden Ansprüche, **dadurch gekennzeichnet, dass** das nicht photokatalytische Metalloxid ein zweiwertiges Metalloxid ist.

6. Verwendung nach Anspruch 5, **dadurch gekennzeichnet, dass** das zweiwertige Metalloxid das Zinkoxid ist.

7. Verwendung nach Anspruch 6, **dadurch gekennzeichnet, dass** das Zinkoxid in Form von sphärischen Partikeln vorliegt, deren mittlerer Durchmesser 1 bis 500 nm beträgt.

8. Verwendung nach einem der vorhergehenden Ansprüche, **dadurch gekennzeichnet, dass** das nicht photokatalytische Metalloxid in einer Menge von 0,5 bis 2 Gew.-%, bezogen auf das Gesamtgewicht der Zusammensetzung, vorliegt.

9. Verwendung nach einem der vorhergehenden Ansprüche, **dadurch gekennzeichnet, dass** das Tocopherol in einer Menge von 0,0005 bis 10 Gew.-% und insbesondere 0,02 bis 6 Gew.-%, bezogen auf das Gesamtgewicht der Zusammensetzung, enthalten ist.

10. Verwendung nach einem der vorhergehenden Ansprüche, **dadurch gekennzeichnet, dass** das Tocopherol ein Gemisch von natürlichen Tocopherolen ist.

11. Verwendung nach einem der vorhergehenden Ansprüche, **dadurch gekennzeichnet, dass** das System außerdem Zusatzstoffe enthält, die einzeln oder im Gemisch verwendet werden und die unter den grenzflächenaktiven Stoffen vom nichtionischen, anionischen, kationischen oder amphoteren Typ, Behandlungsmitteln, Wirkstoffen, Verdickungsmitteln, Suspendiermitteln, Farbmitteln, Parfums, Füllstoffen, Neutralisationsmitteln, Exzipientien und Konservierungsmitteln ausgewählt sind.

12. Verwendung nach Anspruch 11, **dadurch gekennzeichnet, dass** der Zusatzstoff ein Wirkstoff gegen Akne ist.

13. Verwendung nach einem der vorhergehenden Ansprüche, **dadurch gekennzeichnet, dass** das System in eine Zusammensetzung eingearbeitet wird, die in Form einer Creme mit einem pH-Wert von 6 bis 7 vorliegt und ein Polymer auf der Basis von Polyacrylamid, C₁₃₋₁₄-Isoparaffin und Laureth-7 enthält.

14. Zusammensetzung, **dadurch kennzeichnet, dass** sie in einem kosmetisch und/ oder dermatologisch akzeptablen Medium mindestens ein Tocopherol, das unter α-Tocopherol, β-Tocopherol, γ-Tocopherol und/oder δ-Tocopherol ausgewählt ist, mindestens ein nicht photokatalytisches Metalloxid und mindestens einen komplexierenden, Metalle inaktivierenden Stoff enthält, der unter den Phosphonsäurederivaten ausgewählt ist.

15. Zusammensetzung nach Anspruch 14, **dadurch gekennzeichnet, dass** der Metalle inaktivierende, komplexierende Stoff in einer Menge von 0,005 bis 0,1 Gew.-%, bezogen auf das Gesamtgewicht der Zusammensetzung, enthalten ist.

16. Zusammensetzung nach einem der Ansprüche 14 bis 15, **dadurch gekennzeichnet, dass** das Phosphonsäurederivat unter Ethylendiamintetra(methylenphosphonsäure), Diethylentriaminpenta(methylenphosphonsäure) und deren Natriumsalzen ausgewählt ist.

17. Zusammensetzung nach einem der Ansprüche 14 bis 16, **dadurch gekennzeichnet, dass** das Gewichtsverhältnis nicht photokatalytisches Metalloxid/Tocopherol im Bereich von 100:1 bis 1:4 und insbesondere 10:1 bis 2:1 ausgewählt ist.

18. Zusammensetzung nach einem der Ansprüche 14 bis 17, **dadurch gekennzeichnet, dass** das nicht photokatalytische Metalloxid ein zweiwertiges Metalloxid ist.

19. Zusammensetzung nach Anspruch 18, **dadurch gekennzeichnet, dass** das zweiwertige Metalloxid das Zinkoxid ist.

20. Zusammensetzung nach Anspruch 19, **dadurch gekennzeichnet, dass** das Zinkoxid in Form von sphärischen Partikeln vorliegt, deren mittlerer Durchmesser 1 bis 500 nm beträgt.

21. Zusammensetzung nach einem der Ansprüche 14 bis 20, **dadurch gekennzeichnet, dass** das nicht photokatalytische Metalloxid in einer Menge von 0,1 bis 5 Gew.-% und insbesondere 0,5 bis 2 Gew.-%, bezogen auf das Gesamtgewicht der Zusammensetzung, enthalten ist.

22. Zusammensetzung nach einem der Ansprüche 14 bis 21, **dadurch gekennzeichnet, dass** das Tocopherol in einer Menge von 0,0005 bis 10 Gew.-% und insbesondere 0,02 bis 6 Gew.-%, bezogen auf das Gesamtgewicht der Zusammensetzung, enthalten ist.

23. Zusammensetzung nach einem der Ansprüche 14 bis 22, **dadurch gekennzeichnet, dass** das Tocopherol ein Gemisch von natürlichen Tocopherolen ist.

24. Zusammensetzung nach einem der Ansprüche 14 bis 23, **dadurch gekennzeichnet, dass** sie ferner Zusatzstoffe enthält, die einzeln oder im Gemisch verwendet werden und die unter den grenzflächenaktiven Stoffen vom nichtionischen, anionischen, kationischen oder amphoteren Typ, Behandlungsmitteln, Wirkstoffen, Verdickungsmitteln, Suspendiermitteln, Farbmitteln, Parfums, Füllstoffen, Neutralisationsmitteln, Exzipientien und Konservierungsmitteln ausgewählt sind.

25. Zusammensetzung nach Anspruch 24, **dadurch gekennzeichnet, dass** der Zusatzstoff ein Wirkstoff gegen Akne ist.

26. Zusammensetzung nach Anspruch 14, **dadurch gekennzeichnet, dass** sie 0,05 bis 2 % eines Gemisches von natürlichen Tocopherolen, 0,5 bis 5 % Zinkoxid und 0,05 bis 0,1 % Natriumsalz von Diethylentriaminpenta(methylenphosphonsäure) enthält.

## Claims

1. Use of a system composed of at least one non-photocatalytic metal oxide, of at least one tocopherol chosen from α-tocopherol, β-tocopherol, γ-tocopherol and/or δ-tocopherol, and of a metal-inactivating complexing agent chosen from phosphonic acid derivatives, for the manufacture of a composition intended for dermatological treatment for combating and/or preventing irritation, inflammation, immunosuppression and/or acne which are induced by light.

2. Use according to Claim 1, **characterized in that** the complexing agent is present in the proportion of 0.005 % to 0.1 % by weight relative to the total weight of the composition.

3. Use according to either of the preceding claims, **characterized in that** the phosphonic acid derivative is chosen from ethylenediaminetetra(methylenephosphonic acid), diethylenetriaminepenta(methylenephosphonic acid) and their sodium salt.

4. Use according to any one of the preceding claims, **characterized in that** the non-photocatalytic metal oxide/tocopherol weight ratio is chosen to be from 100:1 to 1:4, in particular from 10:1 to 2:1.

5. Use according to any one of the preceding claims, **characterized in that** the non-photocatalytic metal oxide is a bivalent metal oxide.

6. Use according to Claim 5, **characterized in that** the bivalent metal oxide is zinc oxide.

7. Use according Claim 6, **characterized in that** the zinc oxide is in the form of spherical particles whose average diameter is from 1 nm to 500 nm.

8. Use according to any one of the preceding claims, **characterized in that** the non-photocatalytic metal oxide is present in the proportion of 0.5% to 2% by weight relative to the total weight of the composition.

9. Use according to any one of the preceding claims, **characterized in that** the tocopherol is present in the proportion of 0.0005% to 10% by weight, in particular 0.02 % to 6 % by weight relative to the total weight of the composition.

10. Use according to any one of the preceding claims, **characterized in that** the tocopherol is a mixture of natural tocopherols.

11. Use according to any one of the preceding claims, **characterized in that** the system contains, in addition, adjuvants, used alone or mixed, chosen from surfactants of the nonionic, anionic, cationic or amphoteric type, treatment agents, active agents, thickeners, suspending agents, colorants, perfumes, fillers, neutralizing agents, excipients and preservatives.

12. Use according to Claim 11, **characterized in that** the adjuvant is an anti-acne agent.

13. Use according to any one of the preceding claims, **characterized in that** the system is incorporated in a composition which takes the form of a cream having a pH value of 6 - 7, comprising a polymer based on polyacrylamide, C₁₃-C₁₄ isoparaffin and laureth-7.

14. Composition **characterized in that** it comprises, in a cosmetically and/or dermatologically acceptable medium, at least one tocopherol chosen from α-tocopherol, β-tocopherol, γ-tocopherol and/or δ-tocopherol, at least one non-photocatalytic metal oxide and at least one metal-inactivating complexing agent chosen from phosphonic acid derivatives.

15. Composition according to Claim 14, **characterized in that** the said metal-inactivating complexing agent is present in the proportion of 0.005 % to 0.1 % by weight relative to the total weight of the composition.

16. Composition according to either of Claims 14 and 15, **characterized in that** the phosphonic acid derivative is chosen from ethylenediaminetetra-(methylenephosphonic acid), diethylenetriaminepenta-(methylenephosphonic acid) and their sodium salt.

17. Composition according to one of Claims 14 to 16, **characterized in that** the non-photocatalytic metal oxide/tocopherol weight ratio is chosen to be from 100:1 to 1:4, in particular from 10:1 to 2:1.

18. Composition according to one of Claims 14 to 17, **characterized in that** the non-photocatalytic metal oxide is a bivalent metal oxide.

19. Composition according to Claim 18, **characterized in that** the bivalent metal oxide is zinc oxide.

20. Composition according to Claim 19, **characterized in that** the zinc oxide is in the form of spherical particles whose average diameter is from 1 nm to 500 nm.

21. Composition according to one of Claims 14 to 20, **characterized in that** the non-photocatalytic metal oxide is present in the proportion of 0.1% to 5% by weight, in particular 0.5% to 2% by weight relative to the total weight of the composition.

22. Composition according to one of Claims 14 to 21, **characterized in that** the tocopherol is present in the proportion of 0.0005% to 10% by weight, in particular 0.02 % to 6 % by weight relative to the total weight of the composition.

23. Composition according to one of Claims 14 to 22, **characterized in that** the tocopherol is a mixture of natural tocopherols.

24. Composition according to one of Claims 14 to 23, **characterized in that** it contains, in addition, adjuvants, used alone or mixed, chosen from surfactants of the nonionic, anionic, cationic or amphoteric type, treatment agents, active agents, thickeners, suspending agents, colorants, perfumes, fillers, neutralizing agents, excipients and preservatives.

25. Composition according to Claim 24, **characterized in that** the adjuvant is an anti-acne agent.

26. Composition according to Claim 14, **characterized in that** it comprises from 0.05% to 2% of a mixture of natural tocopherols, from 0.5% to 5% of zinc oxide, and from 0.05% to 0.1% of diethylenetriaminepenta(methylenephosphonic acid) sodium salt.
